# EUROPÄISCHE PATENTSCHRIFT

(11) **EP 0 792 440 B1**
(45) Veröffentlichungstag und Bekanntmachung des Hinweises auf die Patenterteilung: **15.09.1999**
(21) Anmeldenummer: 95939255.6
(22) Anmeldetag: 10.11.1995
(51) Int. Cl.: G01D 9/00, G01N 33/48, A61B 5/00

(54) **Blutprobenanalysengerät mit Chipkartenspeicher**
Blood sample analyzing apparatus with chip card memory
Appareil d'analyse de prise de sang avec une mémoire à carte à puce

(30) Priorität: 15.11.1994 DE 9418099 U
(43) Veröffentlichungstag der Anmeldung: 03.09.1997
(73) Patentinhaber: KLOTH, Bernd, 22926 Ahrensburg (DE)
(72) Erfinder: KLOTH, Bernd, 22926 Ahrensburg (DE)
(74) Vertreter: Richter, Werdermann & Gerbaulet
(86) Internationale Anmeldenummer: EP9504427
(87) Internationale Veröffentlichungsnummer: WO9615425

(56) Entgegenhaltungen:
- EP-A- 0 634 659
- US-A- 5 267 152
- US-A- 5 341 291

## Beschreibung

### Anwendungsgebiet

Das erfindungsgemäße Blutprobenanalysengerät ist überall dort besonders vorteilhaft einsetzbar, wo Meßwerte in beliebiger Menge gespeichert und in einfacher Weise an Dritte übermittelt werden sollen.

Die Erfindung geht aus von einem Blutprobenanalysengerät, das über eine Meßeinrichtung, eine Auswertungseinheit mit einem Speicher mit einer Kalibrierkurve sowie eine Anzeige für den Meßwert verfügt.

### Stand der Technik

Bei der Behandlung bestimmter Krankheitsbilder, wie z.B. Hämophilie oder Diabetes mellitus sowie bei Personen, die z.B. Herzklappenträger sind und/oder mit Marcumar behandelt werden, ist die ständige Kontrolle von Blutwerten notwendig, um die mitunter lebenswichtige Einstellung dieser Werte auf den Normbereich zu gewährleisten, gegebenenfalls durch Gabe von Medikamenten.

Üblicherweise werden derartige Analysen, wie Messung der Blutgerinnungszeit oder dgl. in der Praxis des behandelnden Arztes oder in anderen diagnostischen Einrichtungen durchgeführt. Zunehmend besteht jedoch die Tendenz, Patienten mit mobilen Analysengeräten auszustatten, um eine lückenlose Überwachung der Werte zu erzielen. Dadurch werden eine Fülle von Meßdaten ermittelt, die für den Patienten im Rahmen seiner Möglichkeiten zur Anpassung der Medikamentation wichtig sind, aber auch insbesondere dem Arzt wichtige Informationen liefern können.

Bekannte Analysengeräte bieten nicht die Möglichkeit, Meßwerte in ausreichendem Maße, d.h. bei täglicher Messung über mehrere Wochen hinweg, zu speichern und sind zudem für Transportschäden anfällig.

Aus der US-PS 5 341 291 ist bereits ein interaktives medizinisches Testgerät bekannt. Mit diesem Gerät kann ein Patient abgefragt werden, wonach die Antworten des Patienten auf einen räumlich abtrennbaren Speicher zur weiteren Auswertung abgelegt werden. Hierzu kann ein ROM mit einem Steuerprogramm vorgesehen werden, mit dem nicht nur die Steuerung sondern auch die Auswertung der Antworten des Patienten möglich ist. Eine Analyse von Patientenproben und eine gesteuerte Auswertung der erhaltenen Daten ist nicht vorgesehen.

Mit der US-A-5 267 152 ist ein tragbares Analysengerät vorgeschlagen worden, mit dem medizinische Analysen durchführbar sind, deren Meßwerte von einem Mikroprozessor verarbeitet und auf einem eigenen digitalen Display angezeigt werden. Ein räumlich abtrennbarer Speicher, mit dem ein vom Analysengerät unabhängiger Datentransport und eine gesteuerte Auswertung der erhaltenen Meßwerte möglich ist, ist hierbei nicht vorgesehen, so daß für den Patienten weiterhin die Notwendigkeit besteht, die erhaltenen und angezeigten Daten aufzuschreiben, wenn diese einer separaten und zeitlich versetzten Auswertung, beispielsweise durch einen Arzt, zugänglich gemacht werden soll.

### Aufgabe, Lösung, Vorteile

Es ist daher Aufgabe der Erfindung, ein Blutprobenanalysengerät zu schaffen, das es dem Benutzer ermöglicht, gespeicherte Meßwerte in einfachster Weise an andere Personen unabhängig vom Blutprobenanalysengerät zu übermitteln.

Diese Aufgabe wird durch die Merkmale des Anspruchs 1 gelöst.

Dazu ist erfindungsgemäß vorgesehen, ein Analysengerät mit einer Meßeinrichtung, z.B. einer photometrischen Meßeinrichtung sowie einer Auswertungseinrichtung zur Verarbeitung der durch die Meßeinrichtung ermittelten Daten in für den Benutzer geeignete Größen, wobei diese über eine geeignete Anzeige dem Benutzer zur Kenntnis gebracht werden, mit einem Speicher für die Meßwerte zu versehen, der räumlich vom Analysengerät selber abgetrennt werden kann.

Diese vorteilhafte Ausgestaltung erlaubt es, die Meßwerte über einen bestimmten Zeitraum im Speicher abzulegen. Da der Speicher abtrennbar ist, kann dieser z.B. bei einem Arztbesuch mitgeführt werden. Der Arzt kann den Speicher an ein korrespondierendes Gerät anschließen und die Daten abrufen und auswerten. Ist der Speicher gefüllt, so kann er ausgewechselt werden, so daß die speicherbare Datenmenge beliebig groß sein kann.

In einer Ausführungsform ist der Speicher als Chipkarte ausgeführt, wie sie auch z.B. als Telefonkarte bekannt ist. Das Analysengerät muß dann über einen entsprechenden Einschub verfügen sowie über die Einrichtung zum Lesen und Schreiben von Daten auf den Speicherchip.

Derartige Einrichtungen müssen auch jeweils bei anderen Speichermedien vorgesehen sein, die beliebig einsetzbar sind, wie z.B. übliche Computerdisketten oder optische Speichermedien.

Allerdings bietet die Chipkarte aufgrund ihrer geringen Größe die einfachste Transportmöglichkeit, da sie in jede Brieftasche paßt.

Die Verwendung einer derartigen Chipkarte in Verbindung mit einem Analysengerät vermeidet Übertragungsfehler und kann auch zur Archivierung von Meßdaten verwendet werden.

Darüber hinaus ist es möglich, auch Daten zur Steuerung des Analysengerätes auf dem Chip abzulegen, so daß Patienten spezifische Messungen durchführen können.

Die Analysenergebnisse werden vorteilhafterweise nach Datum und Uhrzeit dokumentiert.

Es ist ebenfalls möglich, über einen an das Analysengerät angeschlossenen Drucker oder durch an einen Drucker anschließbares Lesegerät, Ausdrucke der gespeicherten Daten zu erhalten. Das Lesegerät kann auch zur Datenfernübertragung an ein Modem ggf. mittels eines Computers angeschlossen sein, woduch eine Fernbetreuung z.B. aus dem Urlaub für den Arzt möglich wird.

Bei bestimmten Krankheitsbildern wie Diabetes mellitus ist je nach Meßwert eine Medikamentengabe mit einer definierten Dosis notwendig. Da viele Patienten jedoch nicht in der Lage sind, ihren Therapieplan selbst ständig zu variieren, kann in den Speicherchip ein Therapieprogramm integriert werden, das automatisch in Abhängigkeit von den Meßwerten einen Dosierungsvorschlag für das Medikament vorgibt.

Für sehbehinderte Patienten kann das Analysengerät mit einem Lautsprecher bzw. einer Sprachausgabeeinheit versehen sein, so daß die Meßwerte akustisch abrufbar sind.

Vorteilhafterweise erlaubt die Ausstattung des Analysengerätes mit einem auswechselbaren Speicher die Benutzung von mehreren Personen gleichzeitig, da jeder Benutzer vor Inbetriebnahme lediglich seine eigene Chipkarte oder dgl. einführen muß. Dies bietet sich besonders in Pflegeeinrichtungen oder Krankenhäusern an.

Weitere vorteilhafte Ausgestaltungen sind in den Unteransprüchen gekennzeichnet.

### Kurze Beschreibung der Zeichnungen

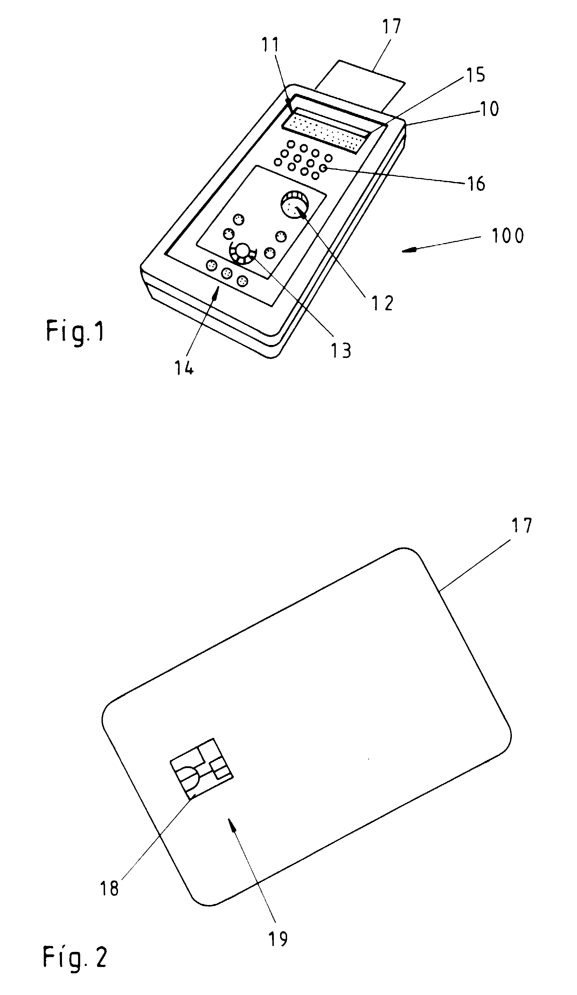

Nachstehend wird die Erfindung anhand von Zeichnungen näher erläutert. Es zeigen
- Fig. 1: in einer perspektivischen Ansicht ein Analysengerät mit Chipkarte als Speicher, und
- Fig. 2.: in einer Draufsicht eine Chipkarte.

### Detaillierte Beschreibung der Erfindung und bester Weg zur Ausführung der Erfindung

Das Analysengerät 100 verfügt über ein Gehäuse 10, das auf der Oberseite 11 eine Öffnung 12 aufweist. In diese Öffnung 12 ist eine nicht dargestellte Meßküvette mit Probenflüssigkeit derart einbringbar, daß sich die Meßküvette im Meßkanal einer im inneren vorgesehenen Meßeinrichtung befindet.

Des weiteren sind auf der Oberseite 11 ein Steuerknopf 13 und Kontrolleuchten vorgesehen. Die Meßwertausgabe erfolgt über eine Anzeige 15, die in üblicher Weise z.B. als Flüssigkristallanzeige ausgestaltet ist, oder über eine akustische Ausgabevorrichtung 16. Der Speicher ist als Chipkarte 17 ausgebildet, die mit dem den Chip 18 tragenden Bereich 19 in eine entsprechende hier nicht sichtbare Öffnung des Analysengerätes 100 reversibel eingesteckt ist. Die im Innenraum vorgesehene Lese- und Schreibvorrichtung für die Chipkarte ist mit einer ebenfalls dargestellten Auswertungseinheit verbunden, die die Daten der Meßeinrichtung in geeignete Größen für die Meßwertausgabe umsetzt.

## Patentansprüche

1. Blutprobenanalysengerät (100), das über eine Meßeinrichtung, eine Auswertungseinheit mit einem Speicher mit einer Kalibrierkurve sowie eine Anzeige (15) für den Meßwert verfügt,
dadurch gekennzeichnet,
daß der Speicher räumlich vom Analysengerät (100) abtrennbar ist, daß der Speicher eine Chipkarte (17) ist und daß der Speicher eine Eichkurve mit reagenzspezifischen Daten, ein Therapieprogramm und ein Programm zur Reagenz-Chargen-Verwaltung beinhaltet.

2. Analysengerät nach Anspruch 1,
dadurch gekennzeichnet,
daß die Anzeige (15) eine LCD-Anzeige ist.

3. Analysengerät nach einem der Ansprüche 1 oder 2,
dadurch gekennzeichnet,
daß das Analysengerät (100) zur Ausgabe von Meßwerten über eine Sprachausgabeeinheit verfügt.

4. Analysengerät nach einem der Ansprüche 1 bis 3,
dadurch gekennzeichnet,
daß an das Analysengerät (100) ein Drucker zur Meßwertausgabe angeschlossen ist.

5. Analysengerät nach einem der Ansprüche 1 bis 4,
dadurch gekennzeichnet,
daß an das Analysengerät (100) ein Bildschirm zur Ausgabe von Meßwerten angeschlossen ist.

6. Analysengerät nach einem der Ansprüche 1 bis 5,
dadurch gekennzeichnet,
daß das Analysengerät (100) an ein Modem zur Datenfernübertragung anschließbar ist, gegebenenfalls mittels eines Computers.

## Claims

1. Blood sample analysis device (100), which comprises a measuring means, an evaluation unit with a memory having a calibration curve as well as a display (15) for the measured data,
characterized in that
the memory is spatially separable from the analysis device (100), in that the memory is a chipcard (17) and in that the memory comprises a calibration curve with reagent-specific data, a therapeutical program and a program for the administration of reagent charges.

2. Analysis device according to Claim 1,
characterized in that
the display (15) is a liquid crystal display.

3. Analysis device according to either Claim 1 or 2,
characterized in that
the analysis device (100) possesses a speech output unit for the output of measured data.

4. Analysis device according to any of Claims 1 through 3,
characterized in that,
to the analysis device (100), a printer is connected for the output of measured data.

5. Analysis device according to any of Claims 1 through 4,
characterized in that,
to the analysis device (100), a video screen is connected for the output of measured data.

6. Analysis device according to any of Claims 1 through 5,
characterized in that
the analysis device (100) is connectable to a modem for the long-distance transmission of data, possibly with the aid of a computer.

## Revendications

1. Appareil d'analyse d'échantillons de sang (100) qui dispose d'un dispositif de mesure, d'une unité d'exploitation avec une mémoire avec une courbe de calibrage ainsi que d'un affichage (15) de la valeur mesurée,
caractérisé en ce
que la mémoire peut être séparée dans l'espace de l'appareil de mesure (100), que la mémoire est une carte à puce (17) et que la mémoire contient une courbe d'étalonnage avec des données spécifiques du réactif, un programme thérapeutique et un programme pour la gestion des charges de réactif.

2. Appareil d'analyse selon la revendication 1,
caractérisé en ce
que l'affichage (15) est un affichage à diodes lumineuses.

3. Appareil d'analyse selon l'une des revendications 1 ou 2,
caractérisé en ce
que l'appareil d'analyse (100) dispose d'une unité de sortie vocale pour la sortie des valeurs mesurées.

4. Appareil d'analyse selon l'une des revendications 1 à 3,
caractérisé en ce
qu'une imprimante est raccordée à l'appareil d'analyse (100) pour la sortie des valeurs mesurées.

5. Appareil d'analyse selon l'une des revendications 1 à 4,
caractérisé en ce
qu'un écran est raccordé à l'appareil d'analyse (100) pour la sortie des valeurs mesurées.

6. Appareil d'analyse selon l'une des revendications 1 à 5,
caractérisé en ce
que l'appareil d'analyse (100) peut être raccordé à un modem pour la transmission à distance des données, le cas échéant au moyen d'un ordinateur.
